# EUROPEAN PATENT SPECIFICATION

(11) **EP 1 729 739 B1**
(45) Date of publication and mention of the grant of the patent: **28.09.2016**
(21) Application number: 05730288.7
(22) Date of filing: 29.03.2005
(51) Int. Cl.: A61K 31/403

(54) **PROCESS FOR PREPARING A SOLID PHARMACEUTICAL COMPOSITION**
VERFAHREN ZUR HERSTELLUNG EINER FESTEN PHARMAZEUTISCHEN ZUSAMMENSETZUNG
PROCEDE DE PREPARATION D'UNE COMPOSITION PHARMACEUTIQUE SOLIDE

(30) Priority: 29.03.2004 DE 102004019845; 09.12.2004 DE 102004059521
(43) Date of publication of application: 13.12.2006
(73) Proprietor: Les Laboratoires Servier, 92284 Suresnes Cedex (FR)
(72) Inventor: KLOBCAR, Iztok, SI-8360 Zuzemberk (SI); PUNCUH-KOLAR, Alesa, SI-8220 Smarjeske Toplice (SI); GRANDOVEC, Anica, SI-8210 Trebnje (SI); TURK, Urska, SI-8000 Novo mesto (SI); SOLMAJER-LAMPIC, Polona, SI-1000 Ljubljana (SI)
(74) Representative: Uexküll & Stolberg
(86) International application number: PCT/EP2005/003277
(87) International publication number: WO 2005/094793

(56) References cited:
- WO-A-03/059388
- WO-A-2005/011737
- GB-A- 2 394 660
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

The invention relates to a process for preparing a solid pharmaceutical composition of perindopril or a salt thereof as well as a solid pharmaceutical composition.

ACE inhibitors, such as Perindopril, are a prodrug for perindo-prilat which is in vivo the actually active substance. Especially solid formulations like tablets suffer from substantial degradation and thereby reduce the effective amount of perindopril. The main degradation routes are 1) the hydrolysis of the ester group and 2) intramolecular cyclization resulting in diketopiperazine (DKP), especially in an acidic environment.

There have been various attempts to stabilize solid compositions of ACE inhibitors.

According to EP-280 999 alkali or alkaline earth metal carbonates have been used to stabilize ACE inhibitor formulations. It is disclosed that in particular magnesium carbonate is a suitable stabilizing carbonate which proves to be effective when combined with enalpril. The components of the compositions are processed by means of wet granulation to the desired tablets.

Further WO 03/075842 disclose formulations of moexipril hydrochloride which have been stabilized by addition of alkali or alkaline earth metal carbonates. A mixture including moexipril hydrochloride as well as the alkaline reacting carbonate is processed by wet granulation so that the stabilizing effect is likely due to the in-situ forming of the sodium salt of moexipril. It is further disclosed that the amount of the carbonate should be greater than the stoichiometric amount of the moexipril hydrochloride.

In the same manner US 5,350,582 discloses the use of stabilizing the ACE inhibitor enalapril maleate by addition of alkaline reacting substances which results in formation of the corresponding more stable sodium salt of enalapril. This in-situ reaction may be accomplished by using sodium hydrogen carbonate and use of a wet granulation process which allows the neutralization between the alkaline stabilizer and the enalapril maleate to occur. For 1 mole of enalapril maleate a total of 3 moles of sodium hydrogen carbonate are used.

However, the afore-mentioned approaches of obtaining stabilized formulations of ACE inhibitors, like perindopril, suffer from the drawback that they always include use of water which in turn can give rise to a reduced stability. Moreover, these processes often do not allow to prepare a pharmaceutical composition which shows a satisfactory level of stability, especially when stored over long periods of time. Finally, the use of a wet granulation step always requires means to remove the granulation liquid at a later stage in order to arrive at the final solid composition.

It is therefore an object of the present invention to provide a process for preparing a solid pharmaceutical composition of perindopril which avoids the above problems of the conventional processes as well as a solid pharmaceutical composition of perindopril which has a high stability and contains only minor amounts of degradation products.

This object is surprisingly achieved by the process according to claims 1 to 12 and the composition according to claims 13 to 17.

The process according to the invention for preparing a solid pharmaceutical composition of perindopril or a salt thereof comprises
(i) dry mixing of perindopril or a salt thereof with at least one inorganic carbonate, at least one carrier, and optionally other components, and
(ii) dry processing of the mixture obtained in step (i) to the desired solid form.

In step (i) perindopril or a salt thereof is dry mixed with at least one inorganic carbonate, at least one carrier and optionally other components. The term "dry mixing" means that to none of the ingredients to be mixed a liquid, like water, ethanol or combinations thereof, is added and additionally that the mixing is effected without adding such a liquid.

Investigations have shown that the perindopril is preferably used in form of its tert.-butylamine salt, which is also referred to as perindopril erbumine, as this leads to particularly stable compositions.

Perindopril erbumine can exist in various polymorphic forms, for example form α disclosed in WO 01/87835, form β disclosed in WO 01/87836 and form γ disclosed in WO 01/83439. It is an advantage of the present composition that an undesired transformation of a polymorph is prevented or at least strongly reduced.

The inorganic carbonate is preferably sodium carbonate, sodium hydrogen carbonate, magnesium carbonate, calcium carbonate, calcium hydrogen carbonate or a mixture thereof.

It has further been shown that particularly stable compositions can be obtained when the molar ratio of perindopril or a salt thereof to inorganic carbonate is 1 to 0.1-0.9 and more preferably 1 to 0.05-0.83.

The carrier can be an inorganic or organic substance. Preferred examples of such carriers are dibasic calcium phosphate, tribasic calcium phosphate, magnesium oxide, microcrystalline cellulose, powdered cellulose, lactose and starch. In a more preferred embodiment the carrier is microcrystalline cellulose, lactose or a mixture thereof.

Particularly preferred is a microcrystalline cellulose which has a low moisture content of 0.3 to 5.0 % by weight, preferably 0.3 to 1.5 % by weight. The moisture content is determined as loss upon drying of a sample in a furnace at 100-150°C until a constant mass is reached.

Additionally, the lactose is particularly preferably anhydrous lactose.

Compositions which have been obtained by using microcrystalline cellulose of the afore-mentioned low moisture content and/or anhydrous lactose show a very low level of degradation and are therefore highly stable products.

Optionally present other components are those conventionally used in the manufacture of pharmaceutical compositions and include for example disintegrants and lubricants. Preferred lubricants may be selected from the group consisting of magnesium stearate, calcium stearate, castor oil, glycerol monostearate, hydrogenated vegetable oil, polyethylene glycol, sodium stearyl fumarate, stearic acid, talc and zinc stearate.

It has further been found particularly preferred that the composition also comprises indapamide or a hydrate thereof. The water content of such a hydrate can vary depending on the humidity level of the atmosphere and can be up to 3 % in case of the hemihydrate. A preferred hydrate is the hemihydrate.

It has also been shown that compositions are preferred which comprise indapamide or a hydrate thereof in form of particles having specific sizes. It is preferred that 90 % by volume of the particles of indapamide or a hydrate thereof have a size of less than 80µm, in particular of less than 70µm.

These preferred particle sizes have a beneficial influence on content uniformity and the release profile of the composition.

In step (ii) the obtained mixture is dry processed to the desired solid form. The term "dry processing" means that no liquid is added to the mixture and that the processing is also effected without addition of any liquid. It is preferred that the mixture obtained in step (i) is processed by means of direct compression using a suitable apparatus, like a punch tableting machine.

Thus, the process according to the invention avoids the use of any liquids, including water or aqueous liquids, which on their own may lead to undesired degradation reactions. It is surprising that despite the avoiding of for example a wet granulation step it is possible by means of the process according to the invention to produce very stable compositions of perindopril or a salt thereof. In particular, it was found that tablets prepared according to the present process, after storage, form only small amounts of diketopiperazine (DKP). It is furthermore surprising that the use of small amounts of inorganic carbonate, i.e. below the stoichiometric amount, provide an additional stabilizing effect even though according to the prior art at least stoichiometric amounts need to be used.

The process according to the invention preferably results in tablets, minitablets or granules.

Further, the invention also relates to a solid pharmaceutical composition of perindopril or salt thereof, comprising
(a) perindopril or a salt thereof,
(b) microcrystalline cellulose having a moisture content of 0.3 to 5.0 % by weight and anhydrous lactose,
(c) at least one inorganic carbonate, wherein the molar ratio of perindopril or a salt thereof to inorganic carbonate is 1 to 0.1-0.9, and
(d) optionally other components.

The preferred embodiments of these composition have already been described above with respect to the process according to the invention. In such a preferred embodiment at least one inorganic carbonate is present in the composition according to the invention. In particular, it is preferred that the molar ratio of perindopril or a salt thereof to inorganic carbonate is 1 to 0.50-0.83.

It is also preferred that the composition further comprises indapamide or a hydrate thereof. It is also preferred that 90 % by volume of the particles of indapamide or a hydrate thereof have a size of less than 80µm, in particular of less than 70µm.

Moreover, the microcrystalline cellulose preferably has a moisture content of 0.3 to 1.5 % weight.

It has surprisingly been shown that by using perindopril or a salt thereof in combination with either microcrystalline cellulose having the specified moisture content of 0.3 to 5.0 % weight and/or anhydrous lactose a very stable composition is obtained. This is in contrast to the teaching of the prior art where the presence of liquid, such as water, is generally required for a wet granulation step or a neutralization reaction between alkaline stabilizer and acidic ACE inhibitor.

Additionally, the present process does not lead to a substantial transformation of polymorphs of perindopril or a salt thereof which is a further benefit in relation to conventional processes.

The following examples serve to illustrate the invention in more detail.

### Examples

### Example 1 (comparison) and Examples 2 to 7

For the examples 2 ,3 (invention) and 4 (reference) perindopril erbumine as well as the materials used as carriers as well as the other components were screened. In examples 5 (invention), 6 (reference) and 7 (invention) the preferred combination of perindopril erbumine with indapamide was used. The screened materials with the exception of the lubricant magnesium stearate were dry blended. Subsequently, the magnesium stearate was added to the resulting mixture and the mixture was homogenized. The homogenized mixture was then compressed using a punch tableting machine, Exacta X of Wilhelm Fette, to give tablets. As a comparison currently marketed tablets containing perindopril erbumine were used having the composition as given in the table below for example 1.

**Table 1**

| Example | 1 (comparison) | 2 | 3 | 4 (reference) | 5 | 6 (reference) | 7 |
|---|---|---|---|---|---|---|---|
| ingredient | mg/ tablet | mg/tablet | mg/tablet | mg/tablet | mg/tablet | mg/tablet | mg/tablet |
| Perindopril erbumine | 4 | 4 | 4 | 4 | 4 | 4 | 4 |
| Indapamide | - | - | - | - | 1.25 | 1.25 | 1.25 |
| Microcrystalline cellulose | 22.50 | 22.50 | - | - | - | - | - |
| Microcrystalline cellulose low moisture content of < 1.5% | - | - | 22.50 | 22.50 | 22.50 | 22.50 | 22.50 |
| Lactose monohydrate | 62.78 | 62.15 | - | 62.15 | 71.03 | 71.53 | 70.78 |
| Lactose anhydrous | - | - | 62.15 | - | - | - | - |
| Sodium hydrogen carbonate | - | 0.63 | 0.63 | - | 0.50 | - | 0.75 |
| Colloidal silica | 0.27 | 0.27 | 0.27 | 0.27 | 0.27 | 0.27 | 0.27 |
| Magnesium stearate | 0.45 | 0.45 | 0.45 | 0.45 | 0.45 | 0.45 | 0.45 |

| | | | | | | | |
|---|---|---|---|---|---|---|---|
| * lactose anhydrous is lactose having a water content of less than 1% by weight, determined by the Karl-Fischer method according to Ph.Eur. 2.5.12. | | | | | | | |

The tablets prepared according to example 1 and according to examples 2, 3 and 4 were stored for 3 weeks at 50°C in closed containers. The results given below show the amount of diketopiperazine after 3 weeks.

| Example | Diketopiperazine (DKP) (%) |
|---|---|
| 1 | 0.49 |
| 2 | 0.06 |
| 3 | 0.07 |
| 4 | 0.16 |

The comparison of example 1 with example 4 shows that even without alkaline reacting carbonate there is a decrease of the quantity of diketopiperazine in case of example 4 using microcrystalline cellulose of a low moisture content. In case of examples 2 and 3 even more decreased amounts of diketopiperazine were determined and these examples include as a stabilizer sodium hydrogen carbonate.

The amount of diketopiperazine was determined with a HPLC method using a Hypersil ODS column, 250mm x 4.6mm i.d., packed with 5µm particles, and a detector operating at a wavelength of 215mm.

A gradient elution was effected using the following mobile phase
A: buffer solution of pH 2.0 prepared by adding into a 1000ml volumetric flask, 0.92g sodium heptansulfonate, and 1ml Triethylamin (TEA) and filling with water to volume, and adjusting pH value of solution to 2.0 with perchloric acid
B: acetonitrile

| **Time (min)** | **%A** | **%B** |
|---|---|---|
| 0 | 70 | 30 |
| 1 | 70 | 30 |
| 20 | 40 | 60 |
| 25 | 40 | 60 |
| 35 | 20 | 80 |
| 40 | 0 | 100 |
| 45 | 70 | 30 |

The flow rate of the mobile phase was set to 1.0 ml/min and the column temperature was set at 70°C. 20µl of a standard solution and of the sample solution at a working concentration of about 3.0 mg/ml of perindopril erbumine in the buffer solution of pH 2.0 were injected. Diketopiperazine was detected on basis the of the retention time of the DKP peak on the chromatogram of the standard solution. The percentage of diketopiperazine was calculated as area %.

Tablets according to examples 2, 3 and 4 were additionally stored for 4 weeks at 40°C in 75% relative humidity in closed containers. Again, the amounts of the degradation product diketopiperazine were determined as mentioned above and the results are given in the table below.

| Example | Diketopiperazine (DKP) (%) |
|---|---|
| 2 | 0.04 |
| 3 | 0.04 |
| 4 | 0.08 |

These experiments showed that a particularly stable composition was obtained in case of examples 2 and 3 which have been prepared without using any wet granulation step and which include sodium hydrogen carbonate as a stabilizer.

Thus, the above experiments show that by suitable selection of excipients and the avoiding of a wet granulation step tablets can be obtained which are, even in the presence of moisture, very stable against degradation.

## Claims

1. Process for preparing a solid pharmaceutical composition of perindopril or a salt thereof, comprising
(i) dry mixing of perindopril or a salt thereof with at least one inorganic carbonate, at least one carrier, and optionally other components, and
(ii) dry processing of the mixture obtained in step (i) to the desired solid form.

2. Process according to claim 1, wherein the composition comprises the tert.-butyl amine salt of perindopril.

3. Process according to claim 1 or 2, wherein the inorganic carbonate is sodium carbonate, sodium hydrogen carbonate, magnesium carbonate, calcium carbonate or calcium hydrogen carbonate.

4. Process according to any one of claims 1 to 3, wherein the molar ratio of perindopril or a salt thereof to inorganic carbonate is 1 to 0.1-0.9 and preferably 1 to 0.50-0.83.

5. Process according to any one of claims 1 to 4, wherein the carrier is microcrystalline cellulose, lactose or a mixture thereof.

6. Process according to claim 5, wherein the microcrystalline cellulose has a moisture content of 0.3 to 5.0 % by weight, preferably 0.3 to 1.5 % by weight.

7. Process according to claim 5 or 6, wherein the lactose is anhydrous lactose.

8. Process according to any one of claims 1 to 7, wherein step (ii) is effected by direct compression of the mixture.

9. Process according to any one of claims 1 to 8, wherein the composition also comprises indapamide or a hydrate thereof.

10. Process according to claim 9, wherein the hydrate is indapamide hemihydrate.

11. Process according to claim 9 or 10, wherein 90 % by volume of the particles of indapamide or a hydrate thereof have a size of less than 80µm.

12. Process according to claim 11, wherein 90 % by volume of the particles of indapamide or a hydrate thereof have a size of less than 70µm.

13. Solid pharmaceutical composition of perindopril or a salt thereof, comprising
(a) perindopril or a salt thereof,
(b) microcrystalline cellulose having a moisture content of 0.3 to 5.0 % by weight and anhydrous lactose,
(c) at least one inorganic carbonate, wherein the molar ratio of perindopril or a salt thereof to inorganic carbonate is 1 to 0.1-0.9, and
(d) optionally other components.

14. Composition according to claim 13, wherein the molar ratio of perindopril or a salt thereof to inorganic carbonate is 1 to 0.50-0.83.

15. Composition according to claim 13 or 14, wherein the microcrystalline cellulose has a moisture content of 0.3 to 1.5 % by weight.

16. Composition according to claim 15 which further comprises indapamide or a hydrate thereof.

17. Composition according to claim 16, wherein 90 % by volume of the particles of indapamide or a hydrate thereof have a size of less than 80µm.

## Patentansprüche

1. Verfahren zur Herstellung einer festen pharmazeutischen Zusammensetzung von Perindopril oder einem Salz davon, bei dem
(i) Perindopril oder ein Salz davon mit mindestens einem anorganischen Carbonat, mindestens einem Träger und gegebenenfalls weiteren Bestandteilen trocken gemischt wird und
(ii) die in Schritt (i) erhaltene Mischung trocken zu der gewünschten festen Form verarbeitet wird.

2. Verfahren nach Anspruch 1, bei dem die Zusammensetzung das tert-Butylaminsalz von Perindopril enthält.

3. Verfahren nach Anspruch 1 oder 2, bei dem das anorganische Carbonat Natriumcarbonat, Natriumhydrogencarbonat, Magnesiumcarbonat, Calciumcarbonat oder Calciumhydrogencarbonat ist.

4. Verfahren nach einem der Ansprüche 1 bis 3, bei dem das molare Verhältnis von Perindopril oder einem Salz davon zu anorganischem Carbonat 1 bis 0,1-0,9 und vorzugsweise 1 bis 0,50-0,83 beträgt.

5. Verfahren nach einem der Ansprüche 1 bis 4, bei dem der Träger mikrokristalline Cellulose, Lactose oder eine Mischung davon ist.

6. Verfahren nach Anspruch 5, bei dem die mikrokrystalline Cellulose einen Feuchtigkeitsgehalt von 0,3-5,0 Gew.-%, vorzugsweise 0,3-1,5 Gew.-% aufweist.

7. Verfahren nach Anspruch 5 oder 6, bei dem die Lactose wasserfreie Lactose ist.

8. Verfahren nach einem der Ansprüche 1 bis 7, bei dem Schritt (ii) durch direkte Verpressung der Mischung bewerkstelligt wird.

9. Verfahren nach einem der Ansprüche 1 bis 8, bei dem die Zusammensetzung auch Indapamid oder ein Hydrat davon enthält.

10. Verfahren nach Anspruch 9, bei dem das Hydrat Indapamidhemihydrat ist.

11. Verfahren nach Anspruch 9 oder 10, bei dem 90 Vol.-% der Teilchen von Indapamid oder einem Hydrat davon eine Größe von weniger als 80 µm aufweisen.

12. Verfahren nach Anspruch 11, bei dem 90 Vol.-% der Teilchen von Indapamid oder einem Hydrat davon eine Größe von weniger als 70 µm aufweisen.

13. Feste pharmazeutische Zusammensetzung von Perindopril oder einem Salz davon, die
(a) Perindopril oder ein Salz davon,
(b) mikrokrystalline Cellulose mit einem Feuchtigkeitsgehalt von 0,3-5,0 Gew.-% und wasserfreie Lactose,
(c) mindestens ein anorganisches Carbonat, wobei das molare Verhältnis von Perindopril oder einem Salz davon zu anorganischem Carbonat 1 bis 0,1-0,9 beträgt, und
(d) gegebenenfalls weitere Bestandteile enthält.

14. Zusammensetzung nach Anspruch 13, bei der das molare Verhältnis von Perindopril oder einem Salz davon zu anorganischem Carbonat 1 bis 0,50-0,83 beträgt.

15. Verfahren nach Anspruch 13 oder 14, bei dem die mikrokrystalline Cellulose einen Feuchtigkeitsgehalt von 0,3-1,5 Gew.-% aufweist.

16. Zusammensetzung nach Anspruch 15, die ferner Indapamid oder ein Hydrat davon enthält.

17. Zusammensetzung nach Anspruch 16, bei dem 90 Vol.-% der Teilchen von Indapamid oder einem Hydrat davon eine Größe von weniger als 80 µm aufweisen.

## Revendications

1. Procédé de préparation d'une composition pharmaceutique solide de périndopril ou d'un sel de périndopril, comportant les étapes suivantes :
i) mélanger à sec du périndopril ou un sel de périndopril avec au moins un carbonate inorganique et au moins un véhicule, et en option, avec d'autres composants,
ii) et traiter à sec le mélange obtenu à l'issue de l'étape (i) pour le mettre sous la forme solide voulue.

2. Procédé conforme à la revendication 1, dans lequel la composition comprend du sel de tertiobutyl-amine de périndopril.

3. Procédé conforme à la revendication 1 ou 2, dans lequel le carbonate inorganique est du carbonate de sodium, de l'hydrogéno-carbonate de sodium, du carbonate de magnésium, du carbonate de calcium, ou de l'hydrogénocarbonate de calcium.

4. Procédé conforme à l'une des revendications 1 à 3, dans lequel le rapport molaire du périndopril ou de son sel au carbonate inorganique vaut de 1/0,1 à 1/0,9 et de préférence de 1/0,50 à 1/0,83.

5. Procédé conforme à l'une des revendications 1 à 4, dans lequel le véhicule est de la cellulose microcristalline, du lactose ou un mélange de ces corps.

6. Procédé conforme à la revendication 5, dans lequel la cellulose microcristalline présente une teneur en humidité de 0,3 à 5,0 % en poids, et de préférence de 0,3 à 1,5 % en poids.

7. Procédé conforme à la revendication 5 ou 6, dans lequel le lactose est du lactose anhydre.

8. Procédé conforme à l'une des revendications 1 à 7, dans lequel l'étape (ii) est effectuée par compression directe du mélange.

9. Procédé conforme à l'une des revendications 1 à 8, dans lequel la composition comprend aussi de l'indapamide ou un hydrate d'indapamide.

10. Procédé conforme à la revendication 9, dans lequel l'hydrate est de l'hémihydrate d'indapamide.

11. Procédé conforme à la revendication 9 ou 10, dans lequel 90 % en volume des particules d'indapamide ou d'hydrate d'indapamide ont une taille inférieure à 80 µm.

12. Procédé conforme à la revendication 11, dans lequel 90 % en volume des particules d'indapamide ou d'hydrate d'indapamide ont une taille inférieure à 70 µm.

13. Composition pharmaceutique solide de périndopril ou d'un sel de périndopril, comprenant :
a) du périndopril ou un sel de périndopril,
b) de la cellulose microcristalline présentant une teneur en humidité de 0,3 à 5,0 % en poids, et du lactose anhydre,
c) au moins un carbonate inorganique, en un rapport molaire du périndopril ou de son sel au carbonate inorganique valant de 1/0,1 à 1/0,9,
d) et en option, d'autres composants.

14. Composition conforme à la revendication 13, dans laquelle le rapport molaire du périndopril ou de son sel au carbonate inorganique vaut de 1/0,50 à 1/0,83.

15. Composition conforme à la revendication 13 ou 14, dans laquelle la cellulose microcristalline présente une teneur en humidité de 0,3 à 1,5 % en poids.

16. Composition conforme à la revendication 15, qui comprend en outre de l'indapamide ou un hydrate d'indapamide.

17. Composition conforme à la revendication 16, dans laquelle 90 % en volume des particules d'indapamide ou d'hydrate d'indapamide ont une taille inférieure à 80 µm.
